# EUROPÄISCHE PATENTSCHRIFT

(11) **EP 1 355 581 B1**
(45) Veröffentlichungstag und Bekanntmachung des Hinweises auf die Patenterteilung: **19.12.2007**
(21) Anmeldenummer: 02702335.7
(22) Anmeldetag: 01.02.2002
(51) Int. Cl.: A61C 8/00, A61B 17/86

(54) **IMPLANTATSYSTEM**
IMPLANT SYSTEM
SYSTEME D'IMPLANT

(30) Priorität: 02.02.2001 DE 10105227
(43) Veröffentlichungstag der Anmeldung: 29.10.2003
(73) Patentinhaber: FRIADENT GmbH, 68229 Mannheim (DE)
(72) Erfinder: HAESSLER, Dieter, 55276 Oppenheim (DE)
(74) Vertreter: Schmitt, Meinrad
(86) Internationale Anmeldenummer: PCT/EP2002/001057
(87) Internationale Veröffentlichungsnummer: WO 2002/062255

(56) Entgegenhaltungen:
- DE-A- 3 800 368
- DE-A- 4 332 075
- US-A- 5 741 267
- US-A- 5 795 160
- US-A- 5 816 812

## Beschreibung

Die Erfindung bezieht sich auf ein Implantatsystem gemäß Patentanspruch 1.

Aus der US 5 795 160 ist ein Implantatsystem mit einem Implantat bekannt, welches einen zervikalen Bereich mit einem Gewinde und daran anschließend einen apikalen, teilweise konisch verjüngt ausgebildeten Bereich aufweist. Ferner ist wenigstens ein Bohrer vorgesehen, um eine gestufte Bohrung in den Knochen zur Aufnahme des Implantats einzubringen. Die gestufte zum apikalen Ende sich verjüngende Bohrung kann gegebenenfalls mit mehreren Bohrern eingebracht werden. Die Durchmesser der Stufen und somit des oder der Bohrer weisen zumindest teilweise einen geringeren Durchmesser als die zugeordneten Bereiche des Implantats derart auf, dass bei der Insertion des Implantats bestimmte Bereiche des Knochens verdichtet werden. Hierbei wird vorausgesetzt, dass das lmplantat zumindest teilweise konisch ausgebildet ist und in Kombination mit der gestuften Bohrung und den Durchmesserdifferenzen die Verdichtung des Knochens erreicht wird.

Ferner ist aus der US 5 741 267 bekannt, Bohrer mit Markierungen zu versehen, und zwar jeweils Bohrer für lmplantate mit unterschiedlichen Durchmessern. Unter Berücksichtigung der Markierungen können, abgestimmt auf den jeweiligen Implantatdurchmesser, in einen Knochen Bohrungen eingebracht werden, deren Lange auf die Implantatlänge abgestimmt ist.

Ferner ist aus der US 5 000 686 ein Implantat bekannt, welches als Dentalimplantat mit einem selbstschneidenden Außengewinde ausgebildet ist. Der Kerndurchmesser des Außengewindes verjüngt sich, ausgehend von einem zervikalen Bereich, hin zu einem apikalen Bereich, wobei im zervikalen Bereich die Gewindetiefe kleiner ist als im apikalen Bereich. Hierdurch wird der Tatsache Rechnung getragen, daß der Knochen im Bereich der Knochenoberkante regelmäßig eine höhere Dichte aufweist, als in den tiefer liegenden Bereichen.

Für unterschiedliche Knochendichten bzw. Knochenqualitäten sind schon Klassifizierungen vorgeschlagen worden, beispielsweise eine Klassifizierung der Qualität des Alveolarknochens in die Klassen DI bis DIV. Die Knochenqualität DI bezeichnet einen sehr kortikalen harten Knochen, während die Knochenqualität DIV einen sehr spongiösen Knochen mit sehr dünner umgebender Kortikalis bezeichnet. Derartige Klassifizierungen der Knochenqualität berücksichtigen die Festigkeit, Größe von Poren, die Kompaktheit, Homogenität, Dichte und dergleichen des Knochens.

Hiervon ausgehend liegt die Erfindung die Aufgabe zugrunde, ein Implantatsystem, insbesondere mit einem Dentalimplantat, vorzuschlagen, welches trotz der unterschiedlichen Ausgangsvoraussetzungen im knöchernen Lager die Erreichung maximaler Primärstabilität des Implantats ermöglicht. Das Implantatsystem soll in einfacher Weise die Insertion des Implantats ermöglichen und in allen Knochenqualitäten höchstmögliche Primärstabilität gewährleisten. Es sollen ein Instrumentarium und ein Implantat vorgeschlagen werden, um in den verschiedenen Knochenqualitäten das Implantat funktionssicher eindrehen zu können und um eine möglichst hohe Primärstabilität zu erhalten.

Die Lösung dieser Aufgabe erfolgt gemäß der in dem Patentanspruch 1 angegebenen Merkmalen.

Das erfindungsgemäße Implantatsystem ermöglicht problemlos die Aufbereitung des knöchernen Lagers mittels der Bohrer und des entsprechend abgestimmten Implantats in Abhängigkeit der Knochenqualität. Die Erfindung ist auf Implantatsysteme beschränkt, deren Primärbohrer im zervikalen Bereich einen Durchmesser aufweisen, der kleiner ist als der Kerndurchmesser des Implantats im zervikalen Bereich, wodurch nach dem Einschrauben des Implantats im Knochen eine verdichtete Zone vorhanden ist. Implantatsysteme, deren Bohrer nicht für eine Knochenverdichtung dimensioniert sind, werden von der Erfindung nicht erfasst. Das Implantat und die Bohrer zur Aufbereitung des Knochenlagers bzw. zur Einbringung der Bohrung sind derart aufeinander abgestimmt, dass insbesondere im zervikalen Knochenbereich beim Einschrauben des Implantats ein interner Kondensationseffekt" erreicht wird. Die axialen und / oder radialen Abmessungen einer beim Einschrauben des Implantats zu verdichtenden Zone im Knochen werden umso größer vorgegeben, je geringer die Knochenqualität ist. Für unterschiedliche Knochenqualitäten wird nicht das Design der Implantate verändert, sondern die Bohrergeometrie und / oder die Einbringtiefe des Bohrers angepasst. Für den harten Knochen im marginalen / zervikalen Bereich ist ein spezieller (Crestal-)Bohrer vorgesehen. Die zu verdichtende Zone liegt im crestalen bzw. zervikalen Knochenbereich, im Bereich der Öffnung in der Knochenoberkante und zwar bevorzugt in einem vorgegebenen Abstand zu dieser. Erfindungsgemäß sind die dem zervikalen Knochenbereich zugeordneten Durchmesser des Gewindekerns und des Bohrers derart aufeinander abgestimmt, dass je weicher der Knochen ist, desto größer die Kondensation des Knochens vorgegeben ist.

Für den Knochen mit größtmöglicher Härte, insbesondere der Knochenqualität Dl, ist die Abstimmung bevorzugt derart, dass eine Kondensation unterbleibt. Beim Einschrauben des Implantats wird insbesondere im subzervikalen Bereich der Knochen verdichtet, wobei auch in einem weicheren Knochen, beispielsweise bei einer Knochenqualität DII, DIII oder DIV, eine hohe Primärstabilität und eine hochwertige Verankerung des Implantats sicher gestellt wird. Andererseits wird erfindungsgemäß eine zu starke Einpressung des Implantats zervikal in den Knochen und eine hieraus resultierende Resorption des Knochens vermieden. Da die interne Kondensation beim Einschrauben des Implantats erfolgt, subkortikal im spongiösen Bereich, werden unerwünschte nachteilige Knochenüberbeanspruchungen, wie zum Beispiel durch Verdichten und über das Klopfen mit entsprechenden geeigneten Handinstrumenten, vermieden. Der Komfort für den Patienten wird durch diese Technik entscheidend verbessert.. Die Größe der zu verdichtenden Zone wird insbesondere durch Vorgabe der axialen Tiefe eines von der Knochenoberkante ausgehenden Knochenbereiches bestimmt. Zusätzlich oder alternativ können der Kerndurchmesser des crestalen oder zervikalen Gewindebereiches und der Bohrungsdurchmesser des zervikalen Knochenbereiches zur Vorgabe der Größe und / oder des Querschnittes der beim Einschrauben des Implantats zu verdichtenden Zone im Knochen in Abhängigkeit der Knochenqualität durch den Zervikal- oder Sekundär-Bohrer vorgegeben werden.

In vorteilhafter Weise ist ein Pilotbohrer zum Einbringen einer initialen Pilotbohrung in den Knochen, insbesondere den Kieferknochen, vorgesehen, wobei die Tiefe der Pilotbohrung im wesentlichen der Länge des Implantats entsprechend vorgegeben wird. Außerdem wird die lmplantatposition und Richtung festgelegt. Insbesondere nach dem Einbringen der Pilotbohrung oder Vorbohrung wird erfindungsgemäß mittels eines Primärbohrers eine Primärbohrung eingebracht, wobei die Vorbohrung einen geringeren Durchmesser als die Primärbohrung aufweist. Der Durchmesser des Primärbohrers und somit der Primärbohrung entspricht zumindest näherungsweise dem Kerndurchmesser im apikalen Endbereich des Implantats. Der apikale Endbereich der Primärbohrung wird nachfolgend auch als erster Bohrungsbereich bezeichnet. Im Rahmen der Erfindung können bei einem beispielsweise konisch sich verjüngenden Gewindekern im zervikalen Bereich des Implantats zumindest für diesen Bereich die Primärbohrung und / oder der hierfür vorgesehene Bohrer entsprechend verjüngt ausgebildet sein. Die Bestimmung der Knochenqualität erfolgt in bevorzugter Weise beim Einbringen der Pilotbohrung und / oder an Hand des ausgebohrten Knochenmaterials. Ferner kann die Bestimmung der Knochenqualität durch geeignete diagnostische Maßnahmen bereits vor dem Einbringen der Vorbohrung oder der Primärbohrung erfolgen, beispielsweise durch Röntgenaufnahmen oder Kernspintomographie. Nachfolgend wird mittels des erfindungsgemäß ausgebildeten Bohrers, der nachfolgend auch als Zervikal- oder Sekundärbohrer bezeichnet wird, eine Sekundärbohrung eingebracht, deren Tiefe in vorteilhafter Weise entsprechend der Knochenqualität vorgegeben wird. Im Falle eines dichten kompakten Knochens, beispielsweise der Knochenqualität Dl, wird eine vergleichsweise große Tiefe vorgegeben, welche zumindest näherungsweise der Länge des zervikalen Bereiches des Implantats entspricht. Die Tiefe der Sekundärbohrung für einen sehr kortikalen harten Knochen ist vorteilhaft zumindest näherungsweise halb so groß wie die Gesamtlänge des Implantats und / oder zumindest näherungsweise gleich groß wie die Tiefe des apikalen Bereichs vorgegeben. Der Sekundärbohrer weist Markierungen zur Vorgabe der Einbringtiefe auf. Diese Markierungen derart ausgebildet, dass das Weiterbohren beendet wird, sobald diese die Oberkante des Knochens erreichen.

Der Durchmesser des Sekundärbohrers und / oder der Sekundärbohrung ist in vorteilhafter Weise derart vorgegeben, dass das Implantat auch in den dichten kompakten Knochen ohne Überbelastung des Knochens problemlos versenkt und / oder eingeschraubt werden kann. Für Knochen mit geringerer Knochenqualität wird die Sekundärbohrung mit geringerer Tiefe eingebracht. Im Grenzfall wird bei einem weichen Knochen, insbesondere entsprechend der Knochenqualität DIV, die Sekundärbohrung nur mit einer derart geringen Tiefe durchgeführt, dass im wesentlichen nur die randständige Kortikalis entfernt wird. Die Kondensationszone beginnt somit in einem vorgegebenen Abstand zur Knochenoberkante. Je "härter" der Knochen ist, desto tiefer wird die Sekundärbohrung durchgeführt bzw. je "weicher" der Knochen ist, desto weniger tief. Anschließend wird das Implantat in die aufbereitete Bohrung des Knochens eingeschraubt, wobei der apikale Gewindebereich des Implantats in den apikalen Bereich der Primärbohrung selbstschneidend eingreift. Da der Durchmesser der Sekundärbohrung dem Kerndurchmesser des zervikalen Bereiches des Implantats entspricht, ist das Implantat in einen harten Knochen problemlos einschraubbar. Bei weichem Knochen erfolgt durch das Einschrauben des im Vergleich zur Bohrung bzw. des Bohrers im zervikalen Bereich des Implantats zweckmäßig erweiterten Gewindekerns in die Zone des nicht ausgebohrten zervikalen Knochenbereiches der interne Kondensationseffekt, so dass der Knochen in der genannten Zone des zervikalen Bereiches verdichtet wird und auch im weichen Knochen erfindungsgemäß die Primärstabilität zur hochwertigen Verankerung des Implantats erreicht wird.

Der Instrumentensatz für die Einbringung der Primärbohrung und der Sekundärbohrung entsprechend der Knochenqualität erfordert somit in vorteilhafter Weise nur zwei Bohrer, wobei der Bohrer für die Sekundärbohrung in zweckmäßiger Weise Mittel, insbesondere Markierungen, entsprechend der einzubringenden Tiefe aufweist. Es sei an dieser Stelle festgehalten, dass Implantate üblicherweise mit unterschiedlichen Durchmessern und / oder Längen bereitgehalten werden. Folglich enthält der Instrumentensatz auf die Durchmesser der Implantate abgestimmte Bohrer, und zwar jeweils einen Bohrer für die Primärbohrung und einen Bohrer für die Sekundärbohrung der jeweiligen Implantatgröße und gegebenenfalls auch einen Vorbohrer. Für Implantate gleichen Durchmessers aber unterschiedlicher Länge gelangt der gleiche Instrumentensatz zum Einsatz, wobei jedoch der Primärbohrer und der zweckmäßigerweise vorgesehene Vorbohrer geeignete Mittel, wie Markierungen oder Stoppelemente, zur Vorgabe der Einbringtiefe entsprechend der Implantatlänge aufweisen. Erfindungsgemäß ist der apikale Gewindebereich insbesondere des Primärbohrers für Implantate gleichen Durchmessers und unterschiedlicher Länge übereinstimmend ausgebildet, wobei die apikalen Gewindebereiche der Implantate gleichfalls übereinstimmend ausgebildet sind.

Gemäß obiger Ausführungen enthält das Implantat ein durchgehendes Gewinde, dessen Kerndurchmesser im zervikalen Bereich größer ist als im apikalen Bereich. Die Gewindetiefe im zervikalen Bereich liegt insbesondere in der Größenordnung zwischen 1/10 und 4/10 mm, und im apikalen Bereich liegt die Gewindetiefe vorteilhaft zwischen 4/10 und 7/10 mm. Im apikalen Bereich des Implantats ist der Gewindekerndurchmesser bevorzugt um einen vorgegebenen Betrag kleiner als im zervikalen Bereich und entspricht insbesondere im wesentlichen dem Durchmesser des Primärbohrers im zweiten bzw. apikalen Bohrerbereich. Die Längen der genannten Gewindebereiche sind zumindest näherungsweise gleich groß vorgegeben, und zwar bevorzugt bei allen Implantaten gleichen Durchmessers aber unterschiedlichen Länge. Für längere Implantate ist erfindungsgemäß noch ein Zwischenbereich vorgesehen, dessen Gewinde im wesentlichen mit dem des apikalen Bereichs übereinstimmt und / oder dessen Gewindekern einen gegenüber dem Durchmesser im zervikalen Bereich um einen vorgegebenen Betrag reduzierten Durchmesser aufweist.

Ferner kann das Implantat im Rahmen der Erfindung auch als eine Stufenschraube ausgebildet sein, welche wenigstens zwei Gewindebereiche oder Stufen mit unterschiedlichen Gewindeaußendurchmessern aufweist, wobei die Stufe am apikalen Ende des Implantats den kleinsten Außendurchmesser besitzt. Derartige Stufenimplantate sind beispielsweise aus dem EP-Patent 0 438 048 bekannt. Entscheidend ist auch für derartige Ausführungsformen, dass insbesondere durch Abstimmung des oder der Durchmesser im zervikalen Bereich des Implantats und des oder der Durchmesser der Bohrung im zervikalen Knochenbereich und / oder der Tiefen der genannten Bereiche in Abhängigkeit von der Knochenqualität beim Einschrauben des Implantats eine interne Kondensation bzw. Verdichtung des Knochens in eine Zone mit vorgebbarer Größe, bevorzugt im subzervikalen Bereich, erreicht wird.

Weiterbildungen und besondere Ausgestaltungen der Erfindung sind in den Unteransprüchen und der nachfolgenden Beschreibung von Ausführungsbeispielen angegeben.

Die Erfindung wird nachfolgend anhand der besonderen Ausführungsbeispiele näher erläutert, ohne dass insoweit eine Beschränkung erfolgt. Es zeigen:
- Figur 1: eine schematische Darstellung der Bohrer und der in den Knochen eingebrachten Bohrungen,
- Figur 2: eine Darstellung mit einem teilweise eingesetzten Implantat,
- Figur 3: eine Darstellung mit dem vollständig eingesetzten Implantat,
- Figur 4: das Implantat teilweise in einer seitlichen Ansicht und teilweise in einer . Axialebene geschnitten,
- Figur 5-12: Schnitte durch Knochen mit vier verschiedenen Knochenqualitäten und die insertierten Implantate.

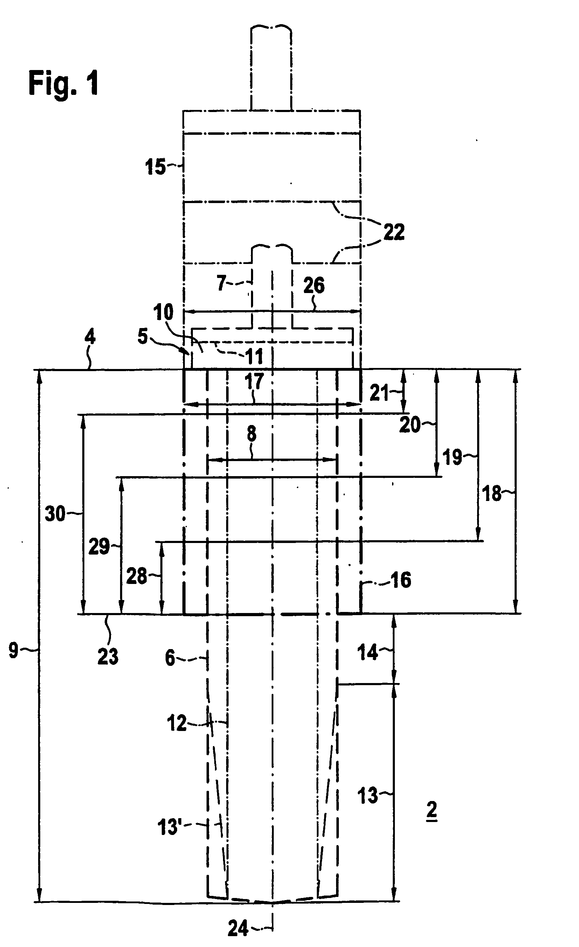

Figur 1 zeigt schematisch einen Schnitt durch einen Knochen 2 mit einer Oberkante 4, in weichen mittels eines Primärbohrers 5 eine mit gestrichelten Linien angedeutete Primärbohrung 6 oder ein erster Bohrungsbereich eingebracht ist. Der gleichfalls mit gestrichelten Linien dargestellte Primärbohrer 5 enthält einen Schaft 7, der in bekannter Weise in ein Bohrgerät einsetzbar ist. Die Primärbohrung 6 oder der erste Bohrungsbereich besitzt einen Durchmesser 8 und eine Länge 9, welche zumindest gleich groß ist wie die Länge des hier nicht dargestellten Implantats. Außerhalb des Knochens 2 bzw. oberhalb der Oberkante 4 weist der Bohrer 5 einen Anschlag oder eine radiale Erweiterung 10 und wenigstens eine Markierung 11 auf, welche Mittel zur Vorgabe der Einbringtiefe der Primärbohrung 6 bilden. Ferner können insbesondere unterhalb der Erweiterung 5 Stoppmittel zur Vorgabe bzw. Begrenzung der Einbringtiefe vorgesehen sein. Mit dem gleichen Primärbohrer 5 können somit in den Knochen 2 auch längere bzw. tiefere Bohrungen eingebracht werden, als gemäß der Länge 9 dargestellt. Der Primärbohrer 5 bzw. die Primärbohrung 6 enthalten einen apikalen Bereich 13, welcher entsprechend dem apikalen Bereich des Implantats ausgebildet ist. Der apikale Endbereich 13 ist für alle Implantatlängen übereinstimmend ausgebildet, während die axiale Länge eines Zwischenbereichs 14 der Primärbohrung 6 entsprechend der Implantatlänge vorgegeben ist. Für Implantate gleichen Durchmessers, jedoch unterschiedlich großer Länge 9, welche bevorzugt im Bereich zwischen 8 bis 20 mm vorgegeben ist, gelangt somit der gleiche Primbohrer 5 zur Verwendung, wobei jedoch die Einbringtiefe in den Knochen 2 vorgegeben wird. Der insbesondere zylindrische Zwischenbereich 14 weist in zweckmäßiger Weise den gleichen Durchmesser 8 auf wie der zervikale Bereich, der in Richtung zur Knochenoberkante 4 anschließt. Insbesondere im apikalen Endbereich 13 kann der Bohrer 5 entsprechend der Ausbildung des Implantats auch eine leicht konische Außenkontur aufweisen, wie es mit gestrichelter Linie 13'angedeutet ist. Wie mit strichpunktierten Linien 12 angedeutet, kann zuvor mittels eines Vorbohrers in den Knochen 2 eine Vorbohrung eingebracht worden sein. Der Durchmesser 8 des Primärbohrers 5 bzw. der Primärbohrung 6 entspricht im wesentlichen dem des Gewindekerns im apikalen Bereich des hier nicht dargestellten Implantats.

Nach dem Einbringen der Primärbohrung 6 wurde mittels eines Sekundärbohrers 15 eine Sekundärbohrung 16 mit einem crestalen zervikalen Durchmesser 17 eingebracht. Erfindungsgemäß ist die Tiefe der Sekundärbohrung 16 und / oder eines korrespondierenden zweiten Bohrungsbereiches der Gesamtbohrung entsprechend der unterschiedlichen Knochenqualitäten, beispielsweise DI bis DIV vorgegeben. Für die Knochenqualität DI, also einen dichten kompakten Knochen, ist die größte Tiefe 18, für die Knochenqualität DII die Tiefe 19, für die Knochenqualität DIII die Tiefe 20 und für die Knochenqualität DIV, also einen sehr spongiösen Knochen mit sehr dünner umgebender Kortikalis, die Tiefe 21 vorgegeben. Der mittels strichpunktierter Linie dargestellte Bohrer bzw. Sekundärbohrer 15 weist bevorzugt Mittel zur Einbringtiefe auf, wobei diese Mittel bevorzugt als in der Außenfläche des Bohrers 15 angeordnete Markierungen 22 ausgebildet sind. Diese Markierungen 22 sind entsprechend den Einbringtiefen 18 bis 21 zueinander beabstandet angeordnet.

Mittels einer Linie 23 ist die Unterkante eines zervikalen Gewindebereiches eines in Richtung der Längsachse 24 in die derart vorbereitete Bohrung des Knochens eingesetzten Implantats angedeutet. Der zervikale Gewindebereich besitzt einen Kerndurchmesser 26, welcher zumindest näherungsweise gleich groß ist wie der Außendurchmesser des Sekundärbohrers 15 bzw. der Innendurchmesser 17 der Sekundärbohrung 16. Die Unterkante entsprechend der Linie 23 des zervikalen Gewindebereiches des Implantats weist nach dessen Insertion zur Knochenoberkante 4 einen Abstand auf, welcher im wesentlichen gleich groß ist wie die Tiefe 18 für die beste Knochenqualität DI. Für diese Knochenqualität kann somit das Implantat in bekannter Weise und / oder mit dem üblicherweise zulässigen Kraftaufwand in den Knochen 2 eingeschraubt werden, wobei die Gewindeflanken des zervikalen Gewindebereiches über die Tiefe 18 erstreckenden Bereich der Sekundärbohrung in den umgebenden zervikalen Knochenbereich einschneiden. Für den Fall einer geringeren Knochenqualität DII wird die Sekundärbohrung nur mit der Tiefe 19 eingebracht, so dass daran apikal die Primärbohrung 6 mit dem Durchmesser 8 anschließt. Zwischen der Unterkante der Primärbohrung mit der Tiefe 19 und der Linie 22 ist somit eine Zone 28 im zervikalen Knochenbereich vorhanden. In dieser Zone 28 wird beim Einschrauben des Implantats das Knochenmaterial durch den zervikalen Gewindekern lateral verdrängt und verdichtet. Analog hierzu werden entsprechend den Tiefen 20, 21 für die Knochenqualitäten DIII und DIV die beim Einschrauben des Implantats zu verdichtenden Zonen des Knochens geschaffen. Für den Fall eines relativ weichen Knochens bzw. für die geringste Knochenqualität DIV wird nur in dem an die Oberkante 4 des Knochens anschließenden Bereich entsprechend der Tiefe 21 mittels des Sekundärbohrers 15 die Primärbohrung auf den Durchmesser 17 erweitert. In dem an die Knochenoberkante 4 anschließenden Bereich mit der Tiefe 21 erfolgt somit keine Kondensation, und ein unerwünschtes Aufreißen des Knochens wird somit zuverlässig vermieden. In diesen Bereich gelangt ein bevorzugt gewindefreier Halsbereich des Implantats mit dem Knochen zur Anlage.

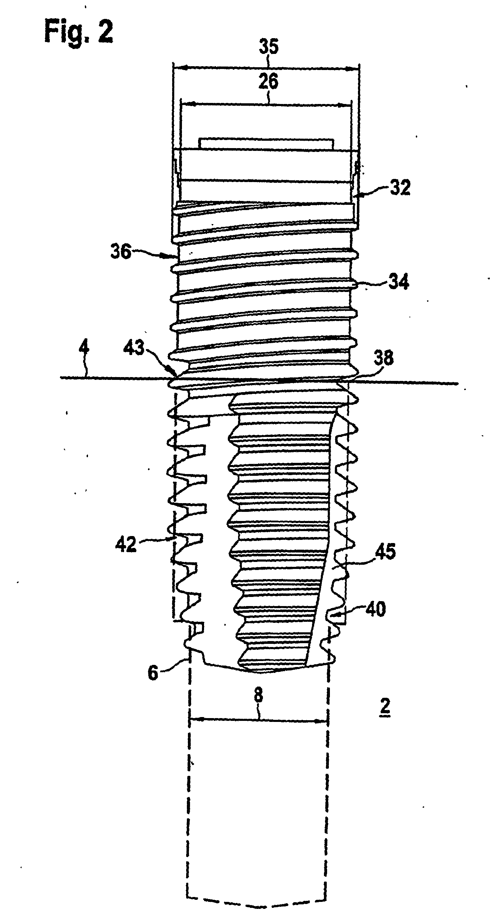

Figur 2 zeigt die derart aufbereitete und an der Oberkante 4 eine Öffnung 38 aufweisende Bohrung mit einem teilweise eingesetzten Implantat 32, welches als Schraubimplantat mit einem selbstschneidenden durchgehenden Außengewinde 34 mit einem Außendurchmesser 35 der Gewindespitzen ausgebildet ist. Das Gewinde 34 des Implantats 32 weist im gemäß Zeichnung oberhalb der Oberkante 4 befindlichen zervikalen Bereich 36 den Kerndurchmesser 26 auf, welcher um einen vorgegebenen Betrag größer ist als der Kerndurchmesser 40 im apikalen Bereich 42. Der Kerndurchmesser 40 ist zumindest näherungsweise gleich groß wie der Durchmesser 8 der Primärbohrung 6. Die Unterkante des zervikalen Bereiches 36 liegt hierbei auf der Höhe der Knochenoberkante 4. Wie ersichtlich, ist das Implantat im apikalen Bereich 42 zum apikalen Ende konisch verjüngt ausgebildet. An den zervikalen Bereich 36 in Richtung zum apikalen Ende anschließend ist ein Übergangsbereich 43 vorgesehen, in welchem der Kerndurchmesser 26 kontinuierlich in den Kerndurchmesser 40 übergeht. Dieser Übergangsbereich 43 erstreckt sich in bevorzugter Weise über wenigstens 360° bzw. über einen Gewindegang, insbesondere über näherungsweise 720° bzw. zwei Gewindegänge. Insbesondere sind maximal drei Gewindegänge vorhanden. Die Länge des Übergangsbereichs 43 ist wesentlich kleiner als die des zervikalen Bereichs 36 und / oder des apikalen Bereichs 42. Somit kann beim Einschrauben des Implantats in die gemäß obigen Ausführungen vorbereitete Bohrung der Knochen mittels des Übergangsbereichs 43 in Gewebe schonender Weise radial verdrängt und verdichtet werden. Wie ersichtlich, weist das Implantat im apikalen Bereich 42 Schneidnuten 44 auf, wobei zweckmäßig drei derartige über den Umfang verteilte Schneidnuten vorgesehen sind. Erfindungsgemäß ist der Kerndurchmesser 26 des Implantatgewindes im zervikalen Bereich 36 um einen vorgegebenen Betrag größer als der Durchmesser 8 der Primärbohrung bzw. des Bohrers, mit welchem jene in den zervikalen Knochenbereich eingebracht worden ist. Beim weiteren Eindrehen des Implantats 32 in den Knochen erfolgt somit aufgrund dieser Durchmesserdifferenz im subzervikalen Bereich des Knochens dessen Kondensation in den oben an Hand von Fig. 1 erläuterten Zonen.

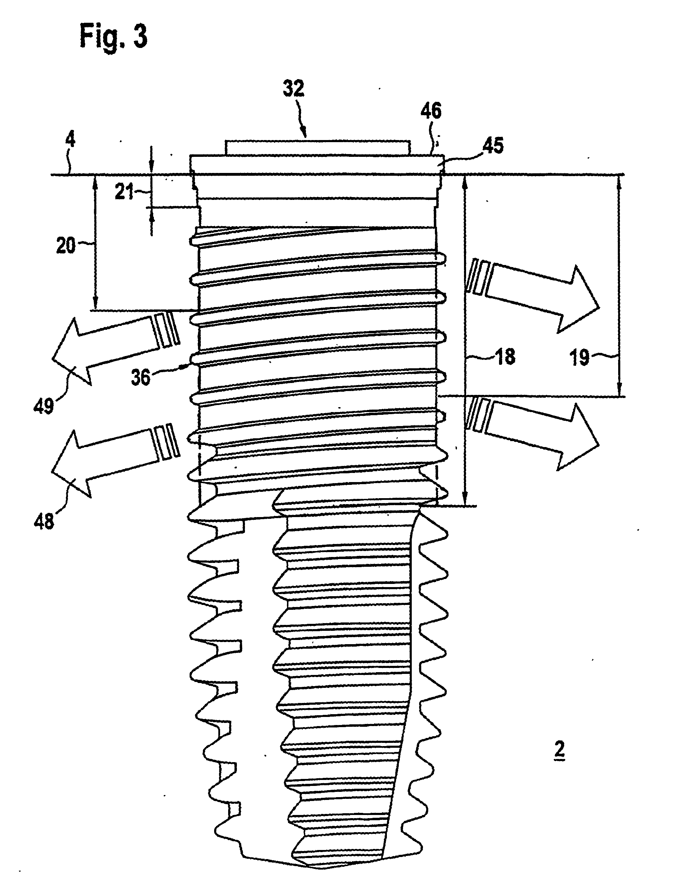

Figur 3 zeigt das vollständig in den Knochen 2 insertierte Implantat 32, dessen bevorzugt gewindefreier Halsbereich 45 die Knochenoberkante 4 durchdringt. Der Halsbereich 45 liegt an der Kortikalis an, wodurch in vorteilhafter Weise eine stabile Verankerung des Implantats in der Kortikalis erreicht wird. Für die Knochenqualität Dl ist gemäß der Tiefe 18 der Sekundärbohrung ein ringförmiger Knochenkern vollständig derart ertfernt, dass das Implantat im wesentlichen frei und problemlos in den Knochen versenkt bzw. eingeschraubt ist, wobei das Gewinde in bekannter Weise in den Knochen eingeschraubt ist. Für weichere Knochenqualitäten entsprechend den Tiefen 19, 20 und 21 wird der dem zervikalen Bereich 36 des Implantats 32 zugeordnete Knochen in den oben erläuterten Zonen nach lateral verdrängt und kondensiert, wie es mittels den Pfeilen 48, 49 angedeutet ist.

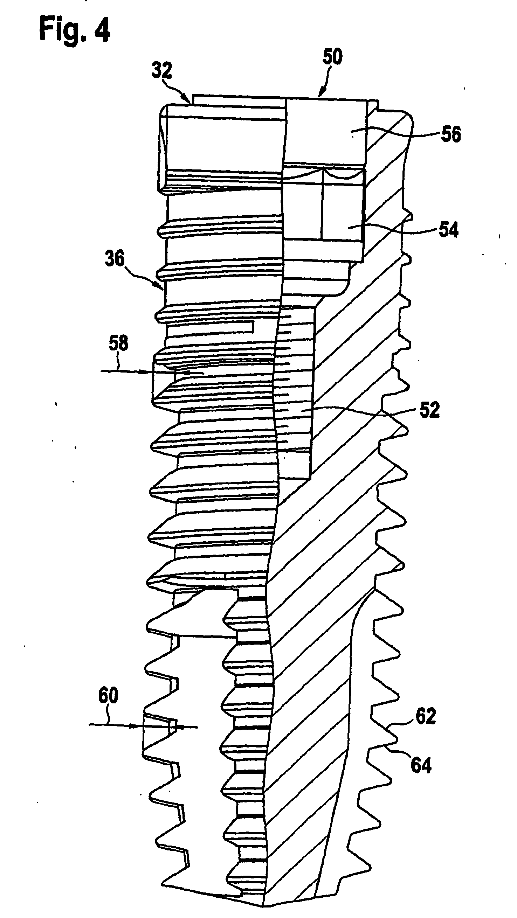

Figur 4 zeigt das Implantat 32 teilweise in einer seitlichen Ansicht und teilweise geschnitten. Das Implantat ist bevorzugt als Dentalimplantat und / oder als Titan-Schraubimplantat ausgebildet und enthält eine Innenbohrung 50 mit einem Innengewinde 52 für eine Halteschraube, mittels welcher in bekannter Weise ein Aufbauteil mit dem Implantat 32 verbindbar ist. Die Bohrung 50 enthält ferner ein Aufnahmemittel 54, insbesondere in Form eines Innensechskants, für ein Werkzeug, mittels welchem das Implantat 32 in den Knochen einschraubbar ist. Des weiteren enthält die Bohrung 50 anschließend an das Plateau 46 eine insbesondere zylindrische Zentrierfläche 56 für das Aufbauelement. Wie ersichtlich, ist im zervikalen Bereich 36 die Gewindetiefe 58 in Richtung zum apikalen Bereich 42 hin zunehmend ausgebildet. Im apikalen Bereich 42 besitzt das Implantat eine Gewindetiefe 60.

In einer besonderen Ausgestaltung wird die Steigung an der Oberseite 62 der Gewindeflanken um einen vorgegebenen Faktor kleiner vorgegeben als die Steigung an der Unterseite 64. So ist die Steigung an der Oberseite 62 insbesondere in der Größenordnung von 1/10 kleiner gewählt als an der Unterseite 64. Somit wird mit zunehmender Höhe das Profil der Gewindeflanke schmaler mit der Folge, dass mit zunehmender Einschraubtiefe der Zuwachs an Friktion im Knochen geringer wird. Hierdurch wird vermieden, dass bei "hartem Knochen" die Friktion zwischen dem Implantat und dem Knochen mit zunehmender Implantatlänge immer weiter ansteigt. So wird die Steigung an der Oberseite 62 insbesondere in der Größenordnung von 0,86 vorgegeben und an der Unterseite 64 in der Größenordnung von 0,87.

Ausführungsbeispiel eines Instrumentensatzes und eines Implantats:

| | |
|---|---|
| Durchmesser des Vorbohrers: | 2,0 mm |
| Durchmesser des Primärbohrers: | 3,5 mm |
| Durchmesser des Sekundärbohrers: | 3,9 mm |
| zervikaler Kerndurchmesser 26 des Implantats: | 3,8 mm |
| apikaler Kerndurchmesser 40 des Implantats: | 3,4 mm |
| Gewindetiefe 58 crestal | 0,2 mm |
| Gewindetiefe 60 apikal: | 0,5 mm |

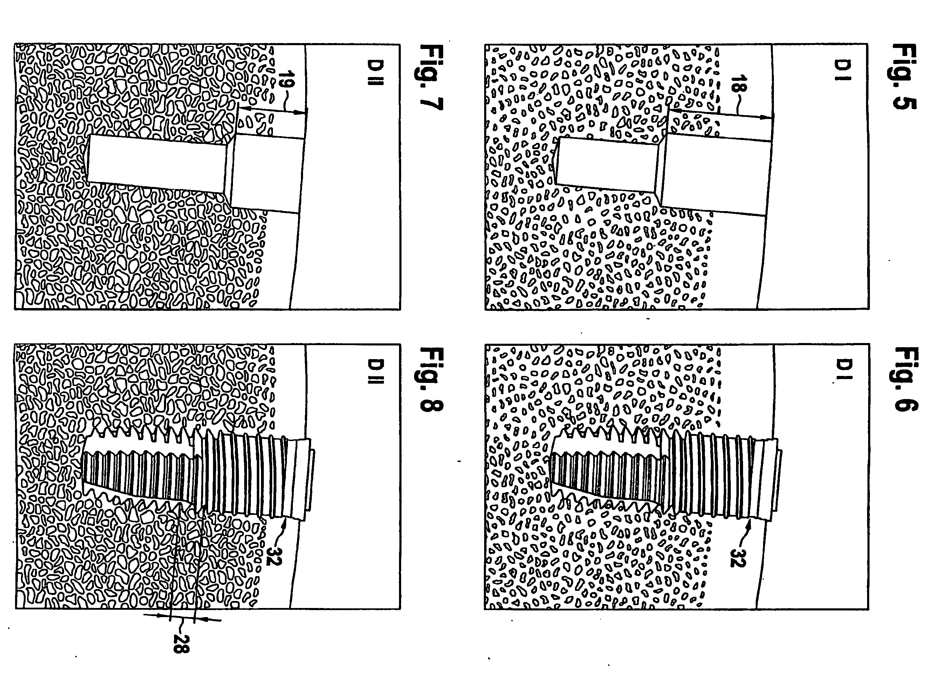

Figur 5 zeigt schematisch einen Schnitt durch einen dichten, kompakten Knochen entsprechend der Knochenqualität DI. Die in diesen Knochen eingebrachte Bohrung weist anschließend an die Oberkante 4 die vergleichsweise große Tiefe 18 auf. Gemäß Figur 6 ist in die derart eingebrachte Bohrung das Implantat 32 eingeschraubt. Wie ersichtlich, ist der zervikale Bereich des Implantats 32 im wesentlichen gleich lang wie die Tiefe 18, so dass das Implantat 32 problemlos und / oder frei in dem dichten und kompakten Knochen eingeschraubt werden konnte.

Figur 7 zeigt schematisch einen porösen kompakten Knochen entsprechend der Knochenqualität DII, wobei im Vergleich mit Figur 5 eine geringere Tiefe 19 für die Sekundärbohrung vorgegeben ist. Gemäß Figur 8 ist In die derart vorbereitete Bohrung wiederum das Implantat 32 eingeschraubt. Da die Länge des zervikalen Bereiches des Implantats 32 größer ist als die Tiefe 19 des zugeordneten Bohrungsteiles, wird somit der Knochen in der Zone 28 im zervikalen Bereich entsprechend verdichtet, so dass trotz reduzierter Knochenqualität eine hohe Primärstabilität gewährleistet ist.

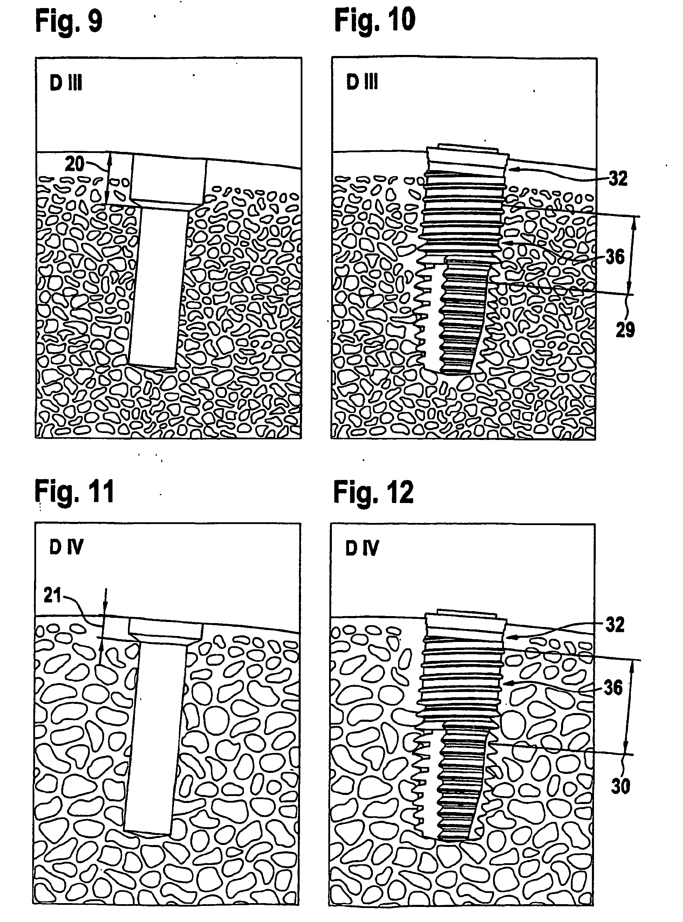

Figur 9 zeigt einen Schnitt durch einen relativ porösen spongiösen Knochen mit vergleichsweise dünner umgebender Kortikalis entsprechend der Knochenqualität DIII. In diesen Knochen ist die Sekundärbohrung mit einer noch geringeren Tiefe 20 eingebracht. Folglich wird gemäß Figur 10 mittels des zervikalen Bereiches 36 des Implantats 32 die im Vergleich mit Figur 8 vergrößerte Zone 29 des Knochens verdichtet.

Schließlich zeigt Figur 11 einen sehr spongiösen Knochen mit sehr dünner umgebender Kortikalis, entsprechend der Knochenqualität DIV, wobei die Tiefe 21 der Sekundärbohrung äußerst gering vorgegeben ist. Dementsprechend erfolgt gemäß Figur 12 praktisch über die gesamte Länge des zervikalen Bereiches 36 des Implantats 32 eine Verdichtung des Knochens in der Zone 30, so dass die geforderte Primärstabilität zur hochwertigen Verankerung des Implantats gewährleistet ist.

Es sei an dieser Stelle ausdrücklich festgehalten, dass in dem Implantatsystem unabhängig von der Knochenqualität immer die gleichen Implantate 32 gleichen Durchmessers zum Einsatz gelangen, wobei durch eine auf die jeweilige Knochenqualität abgestimmte Vorgabe der Tiefe der Sekundärbohrung und / oder der Größe der insbesondere dem zervikalen Implantatbereich zugeordneten Zonen die hohe Primärstabilität gewährleistet wird. Des weiteren gelangen für Implantate 32 gleichen Durchmessers, aber unterschiedlicher Länge, jeweils die gleichen Bohrer zum Einsatz. Es versteht sich, dass das Implantatsystem für Implantate mit anderen Durchmessern darüber hinaus Bohrer mit entsprechend abgestimmten Durchmessern aufweist. So umfasst beispielsweise ein komplettes Implantatsystem für Implantate mit unterschiedlichen Durchmessern von beispielsweise 3 mm, 3,4 mm, 3,8 mm, 4,5 mm und 5,5 mm jeweils einen zugeordneten Bohrersatz, insbesondere aus Vorbohrer, Primärbohrer und Sekundärbohrer, mittels welchen die Bohrungen in den Knochen für Implantate der jeweiligen Durchmesser aber unterschiedlicher Länge gemäß obigen Ausführungen in den Knochen eingebracht werden.

### Bezugszeichen

- 2: Knochen
- 4: Oberkante von 2
- 5: Primärbohrer
- 6: Primärbohrung / erster Bohrungsbereich
- 7: Schaft von 5
- 8: Durchmesser von 5 oder 6
- 9: Länge von 5 oder 6
- 10: Anschlag / Erweiterung von 5
- 11: Markierung
- 12: strichpunktierte Linie / Vorbohrung
- 13: apikaler Endbereich
- 14: Zwischenbereich von 6
- 15: Sekundärbohrer
- 16: Sekundärbohrung / zweiter Bohrungsbereich
- 17: crestaler Durchmesser von 16
- 18-21: Tiefen von 16
- 22: Mittel / Markierung
- 23: Linie
- 24: Längsachse
- 26: Kerndurchmesser des lmplantatgewindes in 36
- 28, 29, 30: zu verdichtende Zone
- 32: Implantat
- 34: Gewinde von 32
- 35: Außendurchmesser von 34
- 36: zervikaler Bereich von 32
- 38: Öffnung
- 40: Kerndurchmesser in 42
- 42: apikaler Bereich von 32
- 43: Übergangsbereich von 26 auf 40
- 44: Schneidnut
- 45: Halsbereich
- 46: Plateau
- 48, 49: Pfeil
- 50: Innenbohrung
- 52: Innengewinde
- 54: Aufnahmemittel
- 56: Zentrierfläche
- 58: Gewindetiefe in 36
- 60: Gewindetiefe in 42
- 62: Oberseite von 34
- 64: Unterseite von 34

## Patentansprüche

1. Implantatsystem, enthaltend einen Primärbohrer (5) und ein Implantat (32), insbesondere ein Dentalimplantat, mit einem zervikalen Bereich (36) und einem apikalen Bereich (42) sowie mit einem bevorzugt selbstschneidenden und einen Gewindekern aufweisenden Gewinde (34), wobei der Primärbohrer (5) zumindest in einem dem zervikalen Bereich (36) des Implantats (32) zugeordneten Bereich einen Durchmesser (8) aufweist, welcher kleiner ist als der Kerndurchmesser des Implantats (32) im zervikalen Bereich (36), wobei nach dem Einschrauben des Implantats im Knochen eine verdichtete Zone (28 - 30) vorhanden ist,
wobei ein Sekundärbohrer (15) vorgesehen ist, dessen Durchmesser (17) einerseits um einen vorgegebenen Betrag größer ist als der Durchmesser (8) des Primärbohrers (5) und andererseitst gleich groß ist wie der Kerndurchmesser des Implantats (32) im zervikalen Bereich (36),
wobei der Sekundärbohrer (15) auf dessen Einbringtiefe (15) in den Knochen entsprechend dessen Knochenqualität abgestimmte und axial beabstandet zueinander angeordnete Markierungen (22), aufweist,
wobei der Außendurchmesser (35) der Zahnspitzen des Gewindes (34) des Implantats (32) im zervikalen Bereich (36) größer ist als der genannte Durchmesser (17) des Sekundärbohrers (15), wobei
das Implantat (32) in Richtung zum apikalen Ende anschließend an den zervikalen Bereich (36) einen Übergangsbereich (43) aufweist, in welchem der Kerndurchmesser (26) kontinuierlich in einen apikalen Gewindebereich (42) mit einem reduzierten Kerndurchmesser (40) übergeht, und wobei der Durchmesser (8) des Primärbohrers gleich groß ist wie der Kerndurchmesser (40) des apikalen Bereiches (42) des Implantats (32).

2. Implantatsystem nach Anspruch 1 **dadurch gekennzeichnet, dass** der Übergangsbereich (43) sich wenigstens um einen Umfangswinkel von 360°, bevorzugt über einen Umfangswinkel von 720°, und / oder über maximal drei Gewindegänge erstreckt.

3. Implantatsystem nach einem der Ansprüche 1 bis 2, **dadurch gekennzeichnet, dass** der Außendurchmesser (35) der Zahnspitzen des Gewindes (34) des Implantats (32) im zervikalen Bereich (36) größer ist als der Durchmesser (17) des Sekundärbohrers (15) oder des zweiten Bohrers.

4. Implantatsystem nach einem der Ansprüche 1 bis 3, **dadurch gekennzeichnet, dass** der Primärbohrer (5) eine Länge aufweist, welche zumindest gleich groß ist wie die in dem Knochen einzubringende Länge des Implantats (32).

5. Implantatsystem nach einem der Ansprüche 1 bis 4, **dadurch gekennzeichnet, dass** für Implantate (32) unterschiedlicher Größe und / oder unterschiedlicher Kerndurchmesser (26) jeweils ein Primärbohrer (5) und ein Sekundärbohrer (15) vorgesehen sind,
wobei bevorzugt ferner ein Vorbohrer jeweils vorgesehen ist, dessen Durchmesser kleiner als der Durchmesser des jeweiligen Primärbohrers (5) ist.

## Claims

1. Implant system, comprising a primary drill (5) and an implant (32), in particular a dental implant with a neck region (36) and an apical region (42), and also a preferably self cutting thread (34) having a thread core, the primary drill (5) having, at least in a region associated with the neck region (36) of the implant (32), a diameter (8) which is smaller than the core diameter of the implant (32) in the neck region (36), a compressed zone (28-30) being present after the implant has been screwed into the bone, wherein a secondary drill (15) is provided, the diameter (17) of which is greater by a specified amount than the diameter (8) of the primary drill (5) on the one hand, and is equal to the core diameter of the implant (32) in the neck region (36) on the other, wherein the secondary drill (15) has markings (22) which are adjusted to the insertion depth (15) of the drill into the bone in accordance with the bone quality thereof and are arranged axially spaced from one another, wherein the outer diameter (35) of the crest of the teeth of the thread (34) of the implant (32) in the neck region (36) is greater than the aforementioned diameter (17) of the secondary drill (15), wherein the implant (32) has a transition region (43) which adjoins the neck region (36) in the direction of the apical end and in which the core diameter (26) changes continuously into an apical thread region (42) with a reduced core diameter (40) and wherein the diameter (8) of the primary drill is equal to the core diameter (40) of the apical region (42) of the implant (32).

2. Implant system according to claim 1, **characterised in that** the transition region (43) extends at least by a peripheral angle of 360°, preferably over a peripheral angle of 720° and/or over three thread flights at most.

3. Implant system according to any one of claims 1 to 2, **characterised in that** the outer diameter (35) of the crest of the teeth of the thread (34) of the implant (32) in the neck region (36) is greater than the diameter (17) of the secondary drill (15) or of the second drill.

4. Implant system according to any one of claims 1 to 3, **characterised in that** the primary drill (5) has a length which is at least equal to the length of the implant (32) to be inserted into the bone.

5. Implant system according to any one of claims 1 to 4, **characterised in that** a primary drill (5) and a secondary drill (15) are each provided for implants (32) of differing sizes and/or differing core diameters (26), a preliminary drill, the diameter of which is smaller than the diameter of the respective primary drills, also preferably being provided in each case.

## Revendications

1. Système d'implant, comprenant un foret primaire (5) et un implant (32), en particulier un implant dentaire, avec une zone cervicale (36) et une zone apicale (42) ainsi qu'un filetage (34) de préférence auto-taraudeur et comprenant une âme de filetage, le foret primaire (5) présentant, au moins dans une zone associée à la zone cervicale (36) de l'implant (32), un diamètre qui est inférieur au diamètre d'âme de l'implant (32) dans la zone cervicale (36), une zone consolidée (28-30) étant présente après le vissage de l'implant dans l'os,
dans lequel un foret secondaire (15) est prévu, dont le diamètre (17) est d'une part plus grand d'une certaine quantité au diamètre (8) du foret primaire (5) et d'autre part est identique au diamètre d'âme de l'implant (32) dans la zone cervicale (36),
dans lequel le foret secondaire (15) présente, sur sa profondeur d'enfoncement (15) dans l'os, des marquages (22) espacés axialement les uns des autres et correspondant à la qualité de l'os,
dans lequel le diamètre extérieur (35) des pointes des dents du filetage (34) de l'implant (32) dans la zone cervicale (36) est plus grand que ledit diamètre (17) du foret secondaire (15),
dans lequel l'implant (32) présente, en direction de l'extrémité apicale, à la suite de la zone cervicale (36), une zone de transition (43) dans laquelle le diamètre (26) d'âme varie continûment jusqu'à une zone apicale de filetage (42) présentant un diamètre d'âme réduit (40), et dans lequel le diamètre (8) du foret primaire est identique au diamètre (40) d'âme de la zone apicale (42) de l'implant (32).

2. Système d'implant selon la revendication 1, ***caractérisé en ce que*** la zone de transition (43) s'étend au moins sur un angle circonférentiel de 360°, de manière préférée sur un angle circonférentiel de 720°, et/ou au plus sur trois filets.

3. Système d'implant selon l'une quelconque des revendications 1 à 2, ***caractérisé en ce que*** le diamètre extérieur (35) des pointes des dents du filetage (34) de l'implant (32) dans la zone cervicale (36) est plus grand que le diamètre (17) du foret secondaire (15) ou du deuxième foret.

4. Système d'implant selon l'une quelconque des revendications 1 à 3, ***caractérisé en ce que*** le foret primaire (5) présente une longueur qui est au moins aussi grande que la longueur de l'implant (32) devant être introduite dans l'os.

5. Système d'implant selon l'une quelconque des revendications 1 à 4, ***caractérisé en ce que**,* pour des implants (32) de différentes tailles et/ou de différents diamètres (26) d'âme il est prévu à chaque fois un foret primaire (5) et un foret secondaire (15), dans lequel de manière préférée il est prévu de plus un pré-foret dont le diamètre est inférieur au diamètre du foret primaire (5) respectif.
